# EUROPEAN PATENT SPECIFICATION

(11) **EP 1 427 427 B1**
(45) Date of publication and mention of the grant of the patent: **08.06.2011**
(21) Application number: 01972468.1
(22) Date of filing: 12.09.2001
(51) Int. Cl.: A61K 31/727, C08B 37/10

(54) **DERIVATIVES OF PARTIALLY DESULPHATED GLYCOSAMINOGLYCANS AS HEPARANASE INHIBITORS, ENDOWED WITH ANTIANGIOGENIC ACTIVITY AND DEVOID OF ANTICOAGULATING EFFECT**
DERIVATE VON TEILDESULFATIERTEN GLYCOSAMINOGLYCANEN ALS INHIBITOREN VON HEPARANASE, MIT ANTIANGIOGENER WIRKUNG UND OHNE ANTIKOAGULATORISCHE WIRKUNG
INHIBITEURS D'HEPARANASE A BASE DE DERIVES DE GLYCOSAMINOGLYCANES PARTIELLEMENT DESULFATES CAPABLES D'ACTIVITE ANTI-ANGIOGENE ET DEPOURVU D'EFFET ANTICOAGULANT

(43) Date of publication of application: 16.06.2004
(60) Divisional application: 10183832.4
(73) Proprietor: SIGMA-TAU Research Switzerland S.A., 6900 Lugano (CH)
(72) Inventor: CASU, B., Ist. Scient. Chim. e Bio. "G. Ronzoni", 20133 Milan (IT); TORRI, G., Ist. Scient. Chim. e Bio. "G. Ronzoni", 20133 Milan (IT); NAGGI, A, Ist. Scient. Chim. e Bio. "G. Ronzoni", 20133 Milan (IT); GIANNINI, Giuseppe, c/o Riunite S.p.A., 00040 Pomezia (IT); PISANO, Claudio, c/o Riunite S.p.A., 00040 Pomezia (IT); PENCO, Sergio, c/o Riunite S.p.A., 00040 Pomezia (IT)
(74) Representative: Spadaro, Marco
(86) International application number: PCT/IT2001/000472
(87) International publication number: WO 2003/022291

(56) References cited:
- EP-A- 0 254 067
- WO-A-01/55221
- WO-A-88/01280
- WO-A-92/01003
- WO-A-94/14851
- WO-A-95/02613
- WO-A-96/06867
- US-A- 5 808 021
- LAPIERRE FRANCE ET AL.: "Chemical modifications of heparin that diminish its anticoagulant but preserve its heparanase-inhibitory, angiostatic, anti-tumor and anti-metastatic properties" GLYCOBIOLOGY, vol. 6, no. 3, 1996, pages 355-366, XP008004538

## Description

The invention described herein relates to partly desulphated heparins, to processes for their preparation, to their use as active ingredients for the preparation of medicaments useful in pathological conditions, like tumors, included the metastatic forms, and for any therapeutic indication gaining benefit from the inhibition of the heparanase, and to pharmaceutical compositions containing them.

### State of the art

Studies performed in the Tumor Biological Research Unit of the Hadassah-Hebrew University Hospital-Israel (Isr. Med. Assoc. J. 2000, 2, 37-45; J. Med. Chem. 2000, 43, 2591-600; Invasion Metastasis 1994-95, 14, 290-302; Exp. Cell Res. 1992, 201, 208-15;) focus on the involvement of heparin-binding growth factors, heparan sulphate and heparan sulphate-degrading enzymes (heparanase) in tumor angiogenesis and metastasis. These studies have been applied to screening and to the identification of heparin derivatives and heparin/heparan sulphate mimetics with potent heparanase inhibiting activity (Nature Med. 1999, 5, 735-6; Science, 1999, 285, 33-4].

Tumor cells release the enzyme heparanase, an endo-β-D-glucuronidase which degrades the polysaccharide chain of heparan sulphate proteoglycans on cell surfaces and in the extracellular matrix.

Involvement in tumor angiogenesis of heparanase has been correlated with the ability to release bFGF (FGF-2) and other growth factors from its storage within the ECM (extracellular matrix). These growth factors provide a mechanism for induction of neovascularization in normal and pathological situations.

Heparanase may thus facilitate not only tumor cell invasion and metastasis but also tumor angiogenesis, both critical steps in tumor progression.

Specific inhibitors of the heparanase enzyme prevent release and activation of growth factors stored by heparan sulphate as well as disruption of the ECM, and are regarded as a very promising approach to develop anticancer drugs.

So, one of possible therapeutic approaches for an antiangiogenic drug is the development of a potent and selective heparanase inhibitor.

For a discussion of angiogenesis, reference may be made to WO 01/55221, in the name of the present applicant.

Another important involvement of heparanase is both inflammation and autoimmunity. In fact, heparanase activity correlates also with the ability of activated cells of the immune system to leave the circulation and elicit both inflammatory and autoimmune responses. Interaction of platelets, granulocytes, T and B lymphocytes, macrophages and mast cells with the subendothelial ECM is associated with degradation of heparan sulphate by heparanase activity. The enzyme is released from intracellular compartments (i.e. lysosomes, specific granules) in response to various activation signals, suggesting its regulated involvement and presence in inflammatory sites and autoimmune lesions. Treatment of experimental animals with heparanase inhibitors (i.e., non-anticoagulant species of low molecular weight heparin - LMWH) markedly reduced the incidence of experimental autoimmune encephalomyelitis (EAE), adjuvant arthritis and graft rejection in experimental animals, indicating that heparanase inhibitors may be applied to inhibit autoimmune and inflammatory disease.

### Heparin

Heparin is a heterogeneous mixture of naturally occurring polysaccharides of various lengths and various degrees of sulphation which possesses anticoagulant activity and is secreted by the connective tissue mast cells present in the liver (from which it was first isolated), in the muscles, lungs, thymus and spleen.

In addition to the regular sequence, a sequence corresponding to the active site for antithrombin activity has been identified in heparin.

The antitumor and antimetastatic activity of heparin and its derivatives is due to its ability to inhibit heparanase, to block growth factors and to regulate angiogenesis.

### Heparan sulphates (HS)

Heparan sulphates (HS) are ubiquitous protein ligands. The proteins bind to the HS chains for a variety of actions from simple immobilisation or protection against the proteolytic degradation action to specific modulations of biological activities, such as angiogenesis.

The carbohydrate skeleton, in both heparin and the heparan sulphates (HS), consists in an alternation of D-glucosamine (GlcN) and hexuronic acids (GlcA or IdoA).

In heparin, the GlcN residues are mainly N-sulphated, whereas in HS they are both N-sulphated and N-acetylated, with a small amount of unsubstituted NH₂ groups.

HS is also on average less O-sulphated than heparin.

The use of heparin in the treatment of angiogenesis disorders, such as tumours, particularly metastases, is substantially limited by the anticoagulant activity of heparin.

Chemical modifications have been made to heparin so as to reduce its anticoagulant capacity, at the same time preserving its antitumor properties.

The opening of a unit of glucuronic acid in the antithrombin site reduces the affinity of heparin for antithrombin: in this way, heparins can be used with reduced anticoagulant effects, but still retaining antiangiogenic properties.

### Heparanases

Heparanases are enzymes belonging to a family of endoglycosidases (an endo-β-D-glucuronidase) that hydrolyse the internal glycoside bonds of the chains of heparan sulphates (HS) and heparin.

These endoglycosidases are involved in the proliferation of tumour cells, in metastases and in the neovascularisation of tumours. These enzymes are biological targets for antiangiogenic activity. In the scientific literature there are a large number of structure/activity correlation studies (see, for example, Lapierre F. et al., Glycobiology, vol. 6, (3), 355-366, 1996). Though many aspects have still to be clarified, studies have been reported regarding the inhibition of heparanases by heparin and its derivatives, using specific tests which have led to the emergence of considerations of a structural type which may serve as guides for obtaining new, more selective derivatives.

In the above-mentioned work of Lapierre et al., heparin derivatives are described as obtained by 2-O desulphation or by "glycol split" (oxidation with periodate and subsequent reduction with sodium borohydride). These derivatives, defined here as "2-O-desulphated heparin" and "RO-heparin", respectively, have partly maintained the antiangiogenic activity of heparin as assessed by means of the CAM test in the presence of corticosteroids (ibid. page 360).

N-acyl heparin derivatives, which are closer mimics of heparan sulphate than heparin, have been reported to inhibit heparanase only somewhat less than N-sulphate derivatives. (Irimira T., Biochemistry 1986, 25, 5322-5328; Ishai-Michaeli R., et al, Biochemistry 1992, 31, 2080-2088).

### Heparins and FGF

FGFs regulate multiple physiological processes such as cell growth and differentiation, but also functions involved in pathological processes such as tumour angiogenesis.

FGFs are growth factors (a family of more than 10 polypeptides, of which the acid (FGF-1) and basic FGFs (FGF-2) are the ones which have been most studied, which require a polysaccharide cofactor, heparin or HS, to bind to the FGF receptor (FGFR) and activate it.

Though the precise mechanism whereby heparin and HS activate FGFs is unknown, it is known, however, that heparin/FGF/FGFR form a "trimolecular" or "ternary" complex.

One mechanism postulated is that heparin and HS induce so-called sandwich dimerisation of FGF, and the latter, thus dimerised, forms a stable complex with FGFR.

### Antimetastatic activity of heparin derivatives

The ability of a primary tumour to generate metastatic cells is perhaps the main problem facing anticancer therapy.

Heparin derivatives with a substantial ability to block heparanase seem to be equally capable of inhibiting angiogenesis both in primary tumours and in metastases.

In addition, the inhibition of heparanase reduces the migration ability of tumour cells from the primary tumour to other organs.

The antimetastatic activity in animal models has been found to correlate with the heparanase-inhibiting ability of heparin and heparin derivatives (Bitan M. et al, Isr. J. Med. Sci. 1995, 31, 106-108) as well as other sulphated polysaccharides (Miao, H. Q. et al, Int. J. Cancer 1999, 83, 424-431, and references therein). Studies on the molecular-weight dependence of the antimetastatic activity indicated that also very low-MW heparins (Sciumbata, T., et al, Invasion Metastasis 1996, 16, 132-143) and oligosaccharide polysulphates (Parish, C.R., et al, Cancer Res. 1999, 59, 3433-3441) retain significant antimetastatic activity. Although in general removal of N-sulphate groups (N-desulphation) decreases the antimetastatic potential of heparins, this activity is partially restored upon N-acylation (N-acetylation, N-hexanoylation (Bitan M., 1995), and N-succinylation (Sciumbata, T., 1996) of resulting free NH₂ groups. The antimetastatic activity of heparins was found to be inversely correlated to their degrees of O-sulphation. (Bitan M., 1995). However, selective 2-O-desulphation of iduronic acid residues did not involve a strong reduction of the antimetastatic activity of heparin (Lapierre, F., Glycobiology 1996, 6, 355-366).

In general, both the heparanase-inhibiting and the antimetastatic activity of heparins and other sulphated polysaccharides decrease with decreasing molecular weight and degree of sulphation (Bitan M., 1995; Parish, C.R., 1999). However, these activities also depend on the carbohydrate backbone of the polysaccharide (type of residues and position of glycosydic linkages) (Parish, C. R., 1999). Since the tridimensional structure of the active site of heparanase is not yet known, it is difficult to predict which polysaccharide backbones and sulphation patterns most effectively inhibit the enzyme.

On the basis of the present knowledge, the structural requirements of heparin-like molecules that favour the angiogenesis-inhibiting action can be grouped in two categories on the basis of the target one intends to block:
a) inhibition of heparanase: although this enzyme recognizes and cleaves heparin and HS sequences of at least eight monosaccharide units containing N-acyl-glucosamine-glucuronic acid (or N-sulphated glucosamine residues see, for example, D. Sandback-Pikas et al. J. Biol. Chem., 273, 18777-18780 (1998) and references cited), its inhibition can be efficiently accomplished by heparin fragments longer than tetradecasaccharide (Bitan M., 1995) or by extensively sulphated, shorter oligosaccharides, such as maltohexaose sulphate (MHS) and phosphomannopentaose sulphate (PI-88) (Parish, C. R., 1999). However, both long heparin fragments and heavily sulphated oligosaccharides are anticoagulant, a property that should be avoided for potential antimetastatic drugs;
b) inhibition of angiogenic growth factors (fibroblast type: FGF-1 and FGF-2; vascular endothelium type: VEGF; vascular permeability type: VPF): to this end the heparin-like compounds preferably have sequences at least five monosaccharide units long, containing 2-sulphated iduronic acid and glucosamine N,6-sulphated (see, for example, M. Maccarana et al. J. Biol. Chem., 268, 23989-23905 (1993)).

The literature discloses small peptides (5-13 amino acids) with antiangiogenic activity (US Patent 5,399,667 of the University of Washington) which act by binding to a thrombospondin receptor, or longer peptides (50 amino acids approx.).

Modified platelet factors are known - (EP 0 589 719, Lilly), capable of inhibiting endothelial proliferation, with IC₅₀=7 nM.

Oligosaccharide fragments with antiangiogenic activity have also been amply described: it has been found, in fact, that by varying the carbohydrate sequence the interaction selectivity can be increased.

In addition, heparin can be used as a vehicle for substances which are themselves antiangiogenic, such as some steroids, exploiting the affinity of heparin for vascular endothelial cells; see, for example, WO 93/18793 of the University of Texas and Imperial Cancer Research Technology, where heparins are claimed with acid-labile linkers, such as adipic acid hydrazine, bound to cortisol. The antiangiogenic effect of the conjugated molecules is greater than that of the unconjugated molecules, even when administered simultaneously.

In Biochim. Biophys. Acta (1996), 1310, 86-96, heparins bound to steroids (e.g. cortisol) are described with a hydrazone group in C-20 that present greater antiangiogenic activity than the two unconconjugated molecules.

EP 0 246 654 by Daiichi Sc. describes sulphated polysaccharides with antiangiogenic activity with Phase II studies. EP 0 394 971 by Pharmacia & Upjohn - Harvard Coll. describes hexa-saccharides - heparin fragments - with low sulphation, capable of inhibiting the growth of endothelial cells and angiogenesis stimulated by FGF-1. EP 0 618 234 by Alfa Wasserman describes a method for preparing semisynthetic glycosaminoglycans with a heparin or heparan structure bearing a nucleophilic group. WO 95/05182 by Glycomed describes various sulphated oligosaccharides with anticoagulant, antiangiogenic and antiinflammatory activity. US 5,808,021 by Glycomed describes a method for preparing substantially non-depolymerised 2-O, 3-O desulphated heparin with a desulphation percentage in positions 2-of the iduronic acid (I, 2-O) and in position 3 of the glucosamine unit (A, 3-O) ranging from approximately 99 to approximately 75% of the original percentage. This method envisages desulphation conducted in the presence of a cation of a bivalent metal, exemplified by calcium or copper, followed by lyophilisation of the product obtained. The desulphated heparins have antiangiogenic activity. EP 0 251 134, Yeda Res & Dev Co Ltd et al, discloses the use of subcoagulant dosages of heparin or its derivatives for preventing allograft rejection and treating autoimmune diseases. The activity of heparin is given by inhibition of heparanase. WO 88/05301, Univ. Australian Nat., discloses antimetastatic and/or antiinflammatory compositions containing a sulphated polysaccharide, which is heparanase inhibitor. Heparin, fucoidan, pentosan sulphate, dextran sulphate are provided. WO 92/01003, Univ. Texas System, discloses the use of a heparin derivative, which is devoid of anticoagulation activity, as heparanase inhibitor. These derivatives have sulphamino or O-sulphate groups, M.W. 1000-15000 and each terminal monomeric unit is a monomeric repeating unit with a terminal O atom bound to a blocking group. WO 94/14851 and WO 96/06867, Glycomed, provide 2-O, 3-O-de-sulphated mucosal heparin, or fragments thereof, being at least 96.7% de-sulphated at the 2-O position and ate least 75% desulphated at the 3-O position useful as non-anticoagulant heparanase inhibitors. WO 95/09637 and WO 96/09828, Glycomed, discloses highly sulphated maltooligosaccharide compounds with heparin like properties. WO 95/30424, Glycomed, provides 6-O-desulphated heparin or fragments thereof with heparanase inhibiting activity. WO 96/33726, Univ. Australian Nat., discloses sulphated oligosaccharides as heparan mimetics having heparanase inhibiting activity. WO 01/35967, Knoll AG, provides a method for treating cardiac insufficiency and related conditions by administering an heparanase inhibitor, among which, heparin which has partly reduced COOH groups, or is at least partly N-desulphated and N-acetylated or is at least partly N,O-desulphated and N-resulphated or is O-acetylated is mentioned.

The aim of the invention described herein is to find optimal glycosaminoglycan structures for generating antiangiogenic activity based on heparanase inhibition and/or FGF growth factor inhibition mechanisms. An additional aim of the invention described herein is to provide a medicament with antiangiogenic activity which is essentially devoid of the typical side effects of heparin derivatives, such as, for example, anticoagulant activity.

WO 01/55221, in the name of the applicant, discloses glycosaminoglycans, particularly a desulphated heparin, with a desulphation degree not greater than 60% of the total uronic units. These derivatives are provided with antiangiogenic activity and are devoid of anticoagulant activity. Said compounds exert their antiangiogenic activity based on the inhibition of FGF. No activity was foreseen for inhibition of heparanase.

In quite general terms, WO 01/55221 also provides a modified heparin, containing glycosamine residues with different degrees of N-desulphation and optional subsequent total or partial acetylation. The general teaching of said reference does not explicitly describe the N-desulphation and optional subsequent total or partial acetylation steps.

### Abstract of the invention

The desulphation carried out in the conditions described in the present invention also produces the formation of iduronic units with an oxyranic ring in position 2,3. The opening of the oxyranic ring in the conditions described in the present invention gives rise to L-iduronic or L-galacturonic units.

It is an object of the invention described herein a modified heparin, containing glycosamine residues with different degrees of N-desulphation and subsequent N-acetylation obtainable by the process disclosed hereinafter.

The compounds which are the subject matter of the invention described herein, are characterized by a high power of inhibiting heparanase with interesting antiangiogenic properties, and are therefore useful as active ingredients for the preparation of medicaments for the treatment of pathologies gaining benefit from the inhibition of the heparanase, pathologies based on abnormal angiogenesis, and particularly for the treatment of metastases.

The compounds according to the present invention also inhibit FGFs.

Advantageously, the compounds according to the present invention show reduced, if not non-existent anticoagulant properties, thus avoiding or reducing the side effects typical of the heparins. A further advantage stems from the fact that the compounds according to the invention can be characterised with instrumental analytical techniques, such as NMR spectroscopy, thus allowing process control which is absolutely desirable from the industrial point of view.

In the modified heparins, molecular weight (MW) has a very important function when making angiogenesis inhibitors. It is well known, in fact, that a reduction in molecular weight (MW) up to values corresponding to penta-saccharide units does not lead to a loss of antiangiogenic activity. On the other hand, it has been established that, whereas beyond a certain length the heparin chains favour rather than inhibit activation of FGF, they are even better inhibitors of heparanase than shorter chains. However, the optimal chain length for inhibition of heparanase depends on the structure of the inhibitor (carbohydrate backbone, positional linkages, sulphation pattern) and should be established for any new type of potential inhibitors.

### Detailed description of the invention

The compounds according to the present invention containing glycosamine residues with different degrees of N-desulphation and subsequent acetylation are herein specifically disclosed and claimed as new compounds.

What is meant by desulphation degree is the percentage of non-sulphated iduronic acids in relation to total uronic acids originally present in the starting heparin. One initial preferred range for the desulphation percentage is from approximately 40 to approximately 60%.

A first preferred compound is a partially N-desulphated and N-reacetylated heparin with a molecular weight (MW) of 11200, a polydispersion index of 1.3, a desulphation degree of 1.6 (expressed as the SO₃⁻: COO⁻ molar ratios, a percentage of modified uronic acids compared to total uronic acids of approximately 30%. and glycosamine residues have a degree of N-acetylation of 50%. Said compound is also called ST1518.

A second preferred compound is an LMW partially N-desulphated and N-reacetylated heparin with a molecular weight of Mn=4780, Mw=10000, a polydispersion index of 2.092, a percentage of modified uronic acids compared, to total uronic acids of approximately 30% and glycosamine residues have a degree of N-acetylation of 50%. Said compound is hereinafter also called ST2168.

A third preferred compound is a partially N-desulphated and N-reacetylated heparin, a percentage of modified uronic acids compared to total uronic acids of approximately 30%. and the glycosamine residues have a degree of acetylation of 27%. This compound is hereinafter also called ST2185.

A fourth preferred compound is a partially N-desulphated and N-reacetylated heparin, a percentage of modified uronic acids compared to total uronic acids of approximately 30%. and the glycosamine residues have a degree of acetylation of 39%. Said compound is hereinafter also called ST2186.

A fifth preferred compound is a partially N-desulphated and N-reacetylated heparin, a percentage of modified uronic acids compared to total uronic acids of approximately 30%. and the glycosamine residues have a degree of N-acetylation of 64%. Said compound is hereinafter also called ST2187.

As far as the N-desulphated and optionally N-acetylated glycosaminoglycans according to the present invention, they can be prepared subjecting heparin to the process, consisting of the following steps:
a) N-desulphation by solvolytic hydrolysis of sulphamino residues in DMSO:H₂O 95:5 v:v at ambient temperature for a time ranging from 0.5 to 8 h, and even more preferably for approximately 2 h, to give the total or partial elimination of sulphate groups at position 2 of the glucosamine residues;
b) N-acetylation of said totally or partially desulphated groups at position 2 of the glucosamine residues by treatment in alkaline aqueous solution (pH 8-9) with an acetylating agent, such as acetyl anhydride, to give totally or partially acetylated groups at position 2 of the glucosamine residues;
c) oxidation of the diols with sodium periodate, to yield the opening of the glycoside ring and the formation of two aldehyde groups per modified residue;
d) reduction of said aldehyde groups to primary alcohol;
e) optional acid hydrolysis of compounds obtained in step d) to obtain oligosaccharides corresponding to the regular sequences, preferably by deamination with nitrous acid. This reaction, which is usually applied to obtain LMW heparin by cleaving the linkage between N-sulphate glucosamine residues and the next uronic acid, leads to a LMW compound having at the non reducing end a residue consisting of an uronic acid and at the reducing end a residue of anhydro mannose, this latter can be further modified to anhydromannitol by reduction with borohydride. The obtained LMW compounds contain at least one residue of glycol-split iduronic acid; or alternatively
f) submitting the products obtained in step d) to partial enzymatic hydrolysis with an enzyme selected from the group consisting of lyase, heparinase, heparitinase, or equivalent of to yield oligosaccharides, preferably tetra- or octa-saccharides, with the non-reducing terminal residue consisting of unsaturated iduronic acid, the reducing residue consisting of an N-sulphoglucosamine and containing at least one residue of open iduronic acid.

The process according to the present invention is also illustrated by the schemes below:

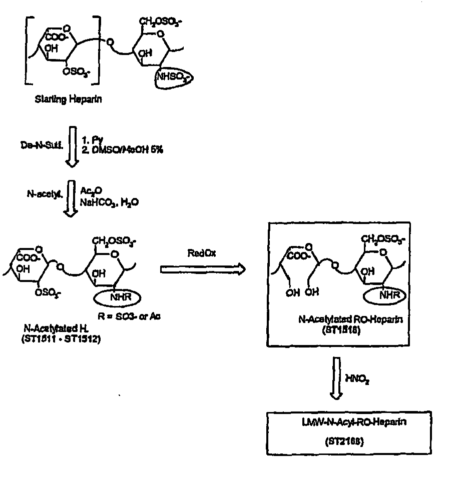

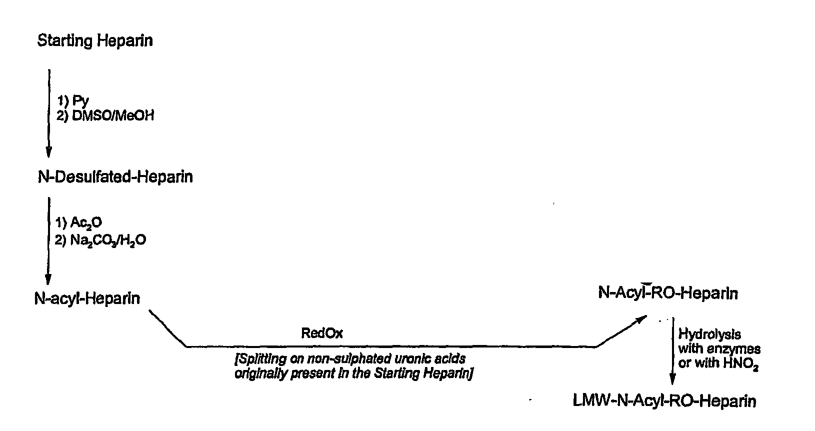

According to the invention described herein, the preferred compound are:
- heparin N-acetyl (50%), obtainable by the process described above, where step a) is conducted for 2h at room temperature and step b) for 2h at 4°C, step c) is conducted at 4°C for one night, step d) for 3 h at room temperature and having a molecular weight (MW) of 11200, a polydispersion index D of 1.3, a desulphation degree of 1.6 (expressed as the SO₃⁻:COO⁻molar ratio), percentage of modified uronic acids compared to total uronic acids of approximately 30% (hereinafter also called ST1518).
- LMW heparin N-acetyl (50%), obtainable by the process described above, where step a) is conducted for 2h at room temperature and step b) for 2h at 4°C, step c) is conducted at 4°C for one night, step d) for 3 h at room temperature, step c) is conducted by nitrous acid deamination at 4°C for 17 min, followed by reduction of aldehyde groups with borohydride at room temperature for 3 h, and having a molecular weight of Mw=4780, Mn= 10000, a polydispersion index D of 2.092, a percentage of modified uronic acids compared to total uronic acids of approximately 30% (hereinafter also called ST2168).

The molecular weights are determined by HPLC-GPC (high performance liquid chromatography - gel permeation chromatography). The desulphation degree is determined by conductimetry and the percentage of modified uronic acids by 13C-NMR.

MW is the molecular weight, and D is the polydispersion index expressed as MW/Mn.

According to the invention described herein, the starting products are naturally occurring heparins. It is also possible to use chemically modified heparins with a percentage content of N,6 disulphate ranging from 0 to 100%. Starting from products with a different 6-O-sulphated glucosamine content, it is possible to modulate the length of the regular sequences between one open iduronic acid and another. The heparins according to the invention that present opening of the glycoside ring are conventionally called RO derivatives by those skilled in the field, meaning by this that the glycoside ring has been opened by means of an oxidation action, followed by a reduction (Reduction-Oxidation - RO). This opening of the glycoside ring is also conventionally called "glycol split", so-called because of the formation of the two primary hydroxy present on the open ring. The compounds referred to herein will also be called "RO" or "Glycol Split" derivatives.

The heparins of the invention can also be used as vehicles for other types of drugs, by means of suitable binding with the heparin portion which is capable of providing a stable bond in normal conditions of manufacture and storage of a formulated drug, which, however, releases the transported drug in the body, preferably in the vicinity of the target organ. Examples of drugs that can be transported are steroidal and non-steroidal antiinflammatory drugs, corticosteroids, and other drugs with an antimetastatic action, in which case there will be an advantageous enhancement of the antimetastatic effect as a result of the sum of the separate intrinsic activities of the compounds according to the invention and the antimetastatic agent bound thereto, with the related advantages of a greater target selectivity and lower systemic toxicity. Examples of these drugs are the metalloproteinase inhibitors. Other drugs which can be usefully transported are those that act at the endothelial level.

In the case of compounds according to the invention deriving from heparin, these are prepared starting from heparin as such by means of N-desulphation followed by N-acylation using techniques known to the technical experts in the field. For example, the N-desulphation is conducted by solvolysis in DMSO:H₂O solution 95:5 v:v at room temperature for time ranging from 0.5 to 8 h followed by N-acylation in alkaline condition with, for example, acylanhydrides (i.e., acetyl, hexanoyl, succinyl, pivaloyl). The heparins of invention can be further degraded with acid agents in suitable pH conditions, e.g. at pH 4, to yield a mixture of oligosaccharides that maintain the antiangiogenic properties.

Objects of the invention described herein are pharmaceutical compositions containing as their active ingredient at least one modified heparin herein disclosed. The active ingredient according to the present invention will be in a mixture with suitable vehicles and/or excipients commonly used in pharmaceutical technology, such as, for instance, those described in "Remington's Pharmaceutical Sciences Handbook", latest edition. The compositions according to the present invention will contain a therapeutically effective quantity of the active ingredient. The doses will be determined by the expert in the field, e.g. the clinician or primary care physician according to the type of disease to be treated and the patient's condition, or concomitantly with the administration of other active ingredients. By way of an example, doses ranging from 0.1 to 100 mg/kg may be indicated.

Examples of pharmaceutical compositions are those that can be administered orally or parenterally, intravenously, intramuscularly, subcutaneously, transdermally or in the form of nasal or oral sprays. Pharmaceutical compositions suitable for the purpose are tablets, hard or soft capsules, powders, solutions, suspensions, syrups, and solid forms for extemporary liquid preparations. Compositions for parenteral administration are, for example, all the intramuscular, intravenous and subcutaneous injectable forms as well as solutions, suspensions and emulsions. Liposome formulations should also be mentioned. The tablets also include forms for the controlled release of the active ingredient whether as oral administration forms, tablets coated with suitable layers, microencapsulated powders, complexes with cyclodextrins, depot forms, for example, subcutaneous forms, such as depot injections or implants.

The compounds according to the invention described herein possess anti-heparanase and antiangiogenic activity. This makes them suitable for the preparation of medicaments useful for the treatment of subjects, generally mammals, and particularly human subjects, suffering from altered angiogenesis or subjects who need a treatment inhibiting the heparanasic activity.

Examples of diseases treated with the medicament which is the object of the present invention are primary tumours, metastases, diabetic retinopathies, psoriasis, retrolenticular fibroplasia, restenosis after angioplasty, coronary by-pass, inflammation, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute glomerulonephritis, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease.

Advantageously, the compounds according to the present invention are substantially devoid of the side effects typical of heparin. In particular, the compounds according to the invention are substantially devoid of anticoagulant activity. By substantially devoid of such activity the expert in the field means no or only negligible activity from the point of view of clinical use.

The heparanase inhibiting activity was determined according to a method established by Vlodavsky's group (Bitan M. et al, 1995). The method is based on evaluation of the extent of fragmentation of the heparan sulphate chains of heparan sulphate proteoglycans (HSPG) caused by heparanase. Sulphate labelled extracellular matrix (ECM) is most commonly used as a source of HSPG. Sulphate labelled ECM is incubated with recombinant heparanase at pH 6.2 in the absence and in the presence of increasing concentrations of the test compound. To evaluate the occurrence of proteoglycan degradation, the incubation medium is collected and applied for gel filtration on Sepharose 6B columns (0.9 x 30 cm). Fractions (0,2 ml) are eluted with PBS at a flow rate of 5 ml/h and counted for radioactivity. The excluded volume (Vₒ) is marked by blue dextran and the total included volume (Vt) by phenol red. Degradation fragments of HS side chains are eluted from Sepharose 6B at 0.5 < Kₐᵥ < 0.8 (peak II). Under the reported experimental conditions, good heparanase inhibitors inhibit fragmentation of HS at concentrations of 10 µg/ml or less.

The results are shown in Table 1, below.

**Table 1**

| Heparanase inhibition at dose ranging from 25 µg/ml to 5 µg/ml | | | | |
|---|---|---|---|---|
| | | **Inhibition** | | |
| | **Dose** | 25 µg/ml | 10 µg/ml | 5 µg/ml |
| | Heparin | 100% | n.d. | >100% |
| ST1516* | Heparin 40 % RO | 100% | n.d. | >85% |
| ST1514* | Heparin~50 % RO | 100% | 100% | >85% |
| ST1515* | Heparin 27.5 % RO | 100% | 100% | 100% |
| ST1518 | 50%NAc heparin 30%RO | 100% | 100% | >85% |

| | | | | |
|---|---|---|---|---|
| * Reference compound disclosed in WO 01/55221 | | | | |

Worthy to be noted, ST1518 has a high inhibition activity even at the concentration of 1 µg/ml.

The compounds according to the present invention, and in particular new one, were tested for their activity with respect to FGF's growth factors, with the same experimental model as described in WO 01/55221 and showed an activity comparable with the ones disclosed in the cited reference.

The following examples further illustrate the invention.

### IEXAMPLE 1

### ST1518

An excess of pyridine was added to an aqueous solution of 1 g of heparin, previously eluted from a column of Amberlite IR 120. The .solution was evaporated under reduced pressure; the resulting pyridine salt of the heparin was dissolved in 50 ml of a mixture of DMSO/H₂O 95:5 and stirred at 20°C for 2 hours, in order to obtain a desulphation degree of about 50%.

Then, the solution was diluted with an equal volume of a saturated solution of NaHCO₃. The solution was dialysed against distilled water in membranes (cut-off 1000-2000D). The final product was isolated by evaporation under reduced pressure.

N-acetylated heparin was prepared by N-acetylation of 50% N-desulphated heparin. 1 g of heparin was dissolved in 10 ml of distilled water; the solution was cooled to 4°C and saturated with sodium hydrogen carbonate; 625 µl of acetic anhydride were added to this solution and the mixture was stirred for 2 hours at 4°C. During the reaction, pH was controlled and maintained at about 8 by adding sodium hydrogen carbonate. Then, the solution obtained was dialysed against distilled water in membranes (cut-off 2000-1000 D).

1 g of heparin 50% N-acetylated heparin is dissolved in 25 ml of distilled water and cooled to 4°C. after the addition of 25 ml of a solution of NaIO₄ 0.2 M, the solution is left to stir in the dark for 20 hours, and the reaction is stopped by adding ethylene glycol and the salts are eliminated by tangential ultrafiltration. 400 mg of NaBH₄, subdivided in several portions, are added to the desalted solution. The solution is left to stir for 3 hours at ambient temperature, then neutralized with diluted HCl and desalted by tangential ultrafiltration.

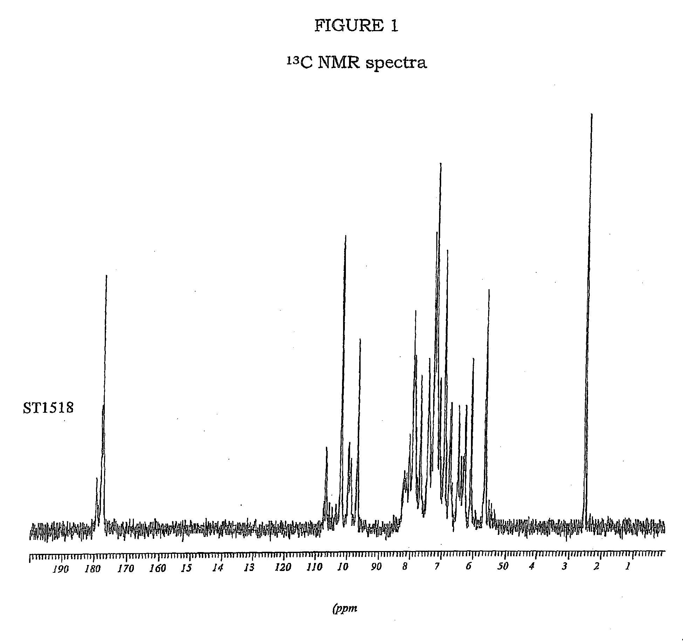

The ¹³C NMR spectrum of the compound is shown in figure 1.

## Claims

1. Modified heparin containing glycosamine residues with different degrees of N-desulphation said subsequent N-acetylation obtainable by subjecting heparin to the process consisting of the following steps:
a) N-desulphation by solvolytic hydrolysis of sulphamino residues in DMSO:H₂O 95:5 v:v at ambient temperature for a time ranging from 0.5 to 8 h, to give the total or partial elimination of sulphate groups at position 2 of the glucosamine residues;
b) N-acetylation of said totally or partially desulphated groups at position 2 of the glucosamine residues by treatment in alkaline aqueous solution (pH 8-9) with an acetylating agent, to give totally or partially acetylated groups at position 2 of the glucosamine residues;
c) oxidation of the diols with sodium periodate, to yield the opening of the glycoside ring and the formation of two aldehyde groups per modified residue;
d) reduction of said aldehyde groups to primary alcohol;
e) optional acid hydrolysis of compounds obtained in step d) to obtain oligosaccharides corresponding to the regular sequences; or alternatively,
f) submitting the products obtained in step d) to partial enzymatic hydrolysis with an enzyme selected from the group consisting of lyase, heparinase, heparitinase, or equivalent of to yield oligosaccharides, with the non-reducing terminal residue consisting of unsaturated iduronic acid, the reducing residue consisting of an N-sulphoglucosamine and containing at least one residue of open iduronic acid.

2. Heparin according to claim 1selected from the group consisting of:
- partially N-desijlphated and N-reacetylated heparin with a molecular weight (MW) of 11200, a polydispersion index of 1.3, a desulphation degree of 1.6 (expressed as SO3-:COO- molar ratio), a percentage of modified uronic acids compared to total uronic acids of approximately 30%, and the glycosamine residues have a degree of N-acetylation of 50%.
- LMW partially N-desulphated and N-reacetylated heparin with a molecular weight of Mn=4780, Mw=10000, a polydispersion index of 2.092, a percentage of modified uronic acids compared to the total uronic acids of approximately 30% and the glycosamine residues have a degree of N-acetylation of 50%,
- partially N-desulphated and N-reacetylated heparin, a percentage of modified uronic acids compared to the total uronic acids of approximately 30%, and the glycosamine residues have a degree of N-acetylation of 27%.
- partially N-desulphated and N-reacetylated heparin, a percentage of modified uronic acid compared to the total uronic acids of approximately 30%, and the glycosamine residues have a degree of N-acetylation of 39%;
- partially N-desulphated and N-reacetylated heparin, a percentage of modified uronic acids compared to the total uronic acids of approximately 30%, and the glycosamine residues have a degree of N-acetylation of 64%.

3. Use of the compounds of claim 1 or 2 for the preparation of a medicament having heparanase inhibiting activity.

4. Use according to claim 3 wherein said medicament has antiangiogenic activity.

5. Use according to any of claims 3-4, wherein said medicament is useful for the treatment of inflammations.

6. Use according to any of claims 3-5, wherein said medicament is useful for the treatment of autoimmune diseases.

7. Use according to any of claims 3-6, wherein the disease to be treated is selected from the group consisting of primary tumours, metastases, diabetic retinopathies, psoriasis, retrolenticular fibroplasia, restenosis after angioplasty, coronary by-pass, inflammation, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute glomerulonephritis, neonatal pulmonary hypertension, asthma, congestive heart failure, adult pulmonary hypertension, renal vascular hypertension, proliferative retinopathies, multiple sclerosis, experimental autoimmune encephalomyelitis, insulin dependent diabetes, inflammatory bowel disease, ulcerative colitis, Crohn's disease.

8. Pharmaceutical composition containing at least one compound of claim 1 or 2 as an active ingredient in admixture with pharmaceutically acceptable vehicles and excipients.

## Patentansprüche

1. Modifiziertes Heparin, welches Glukosamin-Reste mit unterschiedlichen Graden von N-Desulfatierung und nachfolgender N-Acetylierung enthält, erhältlich **dadurch**, dass man Heparin dem Verfahren aussetzt, welches die folgenden Schritte umfasst:
a) N-Desulfatierung durch solvolytische Hydrolyse von Sulfaminoresten in DMSO : H₂O 95 : 5 V : V, bei Umgebungstemperatur einen Zeitraum lang, der von 0,5 bis 8 Stunden reicht, um die vollständige oder Teileliminierung von Sulfatgruppen an Position 2 der Glukosamin-Reste zu erreichen;
b) N-Acetylierung der genannten vollständig oder teilweise desulfatierten Gruppen an Position 2 der Glucosamin-Reste durch Behandeln in alkalischer wässriger Lösung (pH 8 - 9) mit einem Acetylierungsmittel, um vollständig oder teilweise acetylierte Gruppen an Position 2 der Glucosamin-Reste zu erhalten;
c) Oxidation der Diole mit Natriumperiodat, um das Öffnen des Glykosid-Rings und die Bildung von zwei Aldehydgruppen pro modifiziertem Rest zu bewirken;
d) Reduzieren der genannten Aldehydgruppen zu primärem Alkohol;
e) optionale saure Hydrolyse von in Schritt d) erhaltenen Verbindungen, um Oligosaccharide zu erhalten, die zu den Stammsequenzen korrespondieren, oder alternativ
f) Unterziehen der in Schritt d) erhaltenen Produkte einer partiellen enzymatischen Hydrolyse mit einem Enzym, ausgewählt aus der Gruppe, bestehend aus Lyase, Heparinase, Heparitinase oder Äquivalenten davon, um Oligosaccharide zu erhalten, bei denen der nicht-reduzierende terminale Rest aus ungesättigter Iduronsäure besteht, wobei der reduzierende Rest aus einem N-Sulfoglucosamin besteht und wenigstens einen Rest offener Iduronsäure enthält.

2. Heparin gemäß Anspruch 1, ausgewählt aus der Gruppe, bestehend aus
- partiell N-desulfatiertem und N-reacetyliertem Heparin mit einem Molekulargewicht (MW) von 11200, einem Polydispersionsindex von 1,3, einem Desulfatierungsgrad von 1,6 (ausgedrückt als SO3 : COO- Molverhältnis), einem Prozentsatz von modifizierten Uronsäuren im Vergleich zu den gesamten Uronsäuren von ungefähr 30 %, und die Glucosamin-Reste haben einen Grad an N-Acetylierung von 50 %;
- partiell N-desulfatiertem und N-reacetyliertem LMW-Heparin mit einem Molekulargewicht Mn=4780, Mw=10000, einem Polydispersionsindex von 2,092, einem Prozentsatz an modifizierten Uronsäuren im Vergleich zu den gesamten Uronsäuren von ungefähr 30 %, und die Glucosamin-Reste haben einen Grad an N-Acetylierung von 50 %;
- partiell N-desulfatiertes und N-reacetyliertes Heparin mit einem Prozentsatz an modifizierten Uronsäuren im Vergleich zu den gesamten Uronsäuren von ungefähr 30 %, und die Glucosamin-Reste haben einen Grad an N-Acetylierung von 27 %;
- partiell N-desulfatiertes und N-reacetyliertes Heparin mit einem Prozentsatz an modifizierten Uronsäuren im Vergleich zu den gesamten Uronsäuren von ungefähr 30 %, und die Glucosamin-Reste haben einen Grad an N-Acetylierung von 39 %;
- partiell N-desulfatiertes und N-reacetyliertes Heparin mit einem Prozentsatz an modifizierten Uronsäuren im Vergleich zu den gesamten Uronsäuren von ungefähr 30 %, und die Glucosamin-Reste haben einen Grad an N-Acetylierung von 64 %.

3. Verwendung der Verbindungen nach Anspruch 1 oder 2 für die Herstellung eines Medikaments, das Heparanase-hemmende Aktivität besitzt.

4. Verwendung nach Anspruch 3, worin das genannte Medikament antiangiogene Aktivität besitzt.

5. Verwendung gemäß einem der Ansprüche 3 - 4, worin das genannte Medikament für die Behandlung von Entzündungen geeignet ist.

6. Verwendung gemäß einem der Ansprüche 3 - 5, worin das genannte Medikament für die Behandlung von Autoimmunkrankheiten geeignet ist.

7. Verwendung gemäß einem der Ansprüche 3 - 6, worin die zu behandelnde Krankheit ausgewählt ist aus der Gruppe, bestehend aus Primärtumoren, Metastasen, diabetischen Retinopathien, Psoriasis, retrolentikulärer Fibroplasie, Restenose nach Angioplastie, aorto-koronarem Bypass, Entzündung, Arthritis, Autoimmunkrankheiten, Allotransplantationsabstoßung, kardiovaskulären Krankheiten, fibroproliferativen Krankheiten, Krankheiten, die durch abnormale Plättchenaggregation hervorgerufen werden, Krankheiten, die durch Proliferation glatten Muskelgewebes hervorgerufen werden, Goodpasture-Syndrom, akuter Glomerulonephritis, neonataler pulmonaler Hypertonie, Asthma, dekompensierter Herzinsuffizienz, adulter pulmonaler Hypertonie, renovaskulärer Hypertonie, proliferativen Retinopathien, Multiple Sklerose, experimenteller autoimmuner Enzephalitis, insulinabhängiger Diabetes, entzündlicher Darmerkrankung, Colitis ulcerose, Morbus Crohn.

8. Pharmazeutische Zusammensetzung, welche wenigstens eine Verbindung nach Anspruch 1 oder 2 als aktiven Bestandteil in Mischung mit pharmazeutisch verträglichen Trägern und Hilfsstoffen enthält.

## Revendications

1. Héparine modifiée contenant des résidus glycosamines ayant différents degrés de N-désulfatation et de N-acétylation subséquente, susceptible d'être obtenue en soumettant de l'héparine au procédé consistant en les étapes suivantes :
a) N-désulfatation par hydrolyse solvolytique de résidus sulfaminos dans DMSO:H₂O 95:5 v:v à température ambiante pendant une durée allant de 0,5 à 8 h, pour obtenir l'élimination totale ou partielle des groupes sulfates en position 2 des résidus glucosamines ;
b) N-acétylation desdits groupes totalement ou partiellement désulfatés en position 2 des résidus glucosamines par traitement dans une solution aqueuse alcaline (pH 8-9) avec un agent acétylant, pour donner des groupes totalement ou partiellement désacétylés en position 2 des résidus glucosamines ;
c) oxydation des diols avec du periodate de sodium, pour obtenir l'ouverture du noyau glycoside et la formation de deux groupes aldéhydes par résidu modifié ;
d) réduction desdits groupes aldéhydes en alcool primaire ;
e) hydrolyse acide facultative des composés obtenus dans l'étape d) pour obtenir des oligosaccharides correspondant aux séquences normales ; ou, en variante,
f) soumission des produits obtenus dans l'étape d) à une hydrolyse enzymatique partielle avec une enzyme choisie dans le groupe constitué par une lyase, une héparinase, une héparitinase ou un équivalent pour donner des oligosaccharides, le résidu terminal non réducteur consistant en acide iduronique insaturé, le résidu réducteur consistant en une N-sulfoglucosamine et contenant au moins un résidu d'acide iduronique ouvert.

2. Héparine selon la revendication 1, choisie dans le groupe constitué par :
- une héparine partiellement N-désulfatée et N-réacétylée ayant un poids moléculaire (MW) de 11200, un indice de polydispersion de 1,3, un degré de désulfatation de 1,6 (exprimé en rapport molaire SO₃-:COO-), un pourcentage d'acides uroniques modifiés par rapport aux acides uroniques totaux d'approximativement 30 %, et les résidus glycosamines ont un degré de N-acétylation de 50 %
- une héparine partiellement N-désulfatée et N-réacétylée de bas MW, ayant un poids moléculaire de Mn = 4780, Mw = 10000, un indice de polydispersion de 2.092, un pourcentage d'acides uroniques modifiés par rapport aux acides uroniques totaux d'approximativement 30 % et les résidus glycosamines ont un degré de N-acétylation de 50 %
- une héparine partiellement N-désulfatée et N-réacétylée ayant un pourcentage d'acides uroniques modifiés par rapport aux acides uroniques totaux d'approximativement 30 % et les résidus glycosamines ont un degré de N-acétylation de 27 %
- une héparine partiellement N-désulfatée et N-réacétylée ayant un pourcentage d'acides uroniques modifiés par rapport aux acides uroniques totaux d'approximativement 30 % et les résidus glycosamines ont un degré de N-acétylation de 39 %
- une héparine partiellement N-désulfatée et N-réacétylée ayant un pourcentage d'acides uroniques modifiés par rapport aux acides uroniques totaux d'approximativement 30 % et les résidus glycosamines ont un degré de N-acétylation de 64 %.

3. Utilisation des composés selon la revendication 1 ou 2 pour la préparation d'un médicament ayant une activité inhibitrice d'héparanase.

4. Utilisation selon la revendication 3, dans laquelle ledit médicament a une activité anti-angiogénique.

5. Utilisation selon l'une quelconque des revendications 3-4, dans laquelle ledit médicament est utile pour le traitement d'inflammations.

6. Utilisation selon l'une quelconque des revendications 3-5, dans laquelle ledit médicament est utile pour le traitement de maladies auto-immunes.

7. Utilisation selon l'une quelconque des revendications 3-6, dans laquelle la maladie à traiter est choisie dans le groupe constitué par les tumeurs primaires, les métastases, les rétinopathies diabétiques, un psoriasis, une fibroplasie rétrolenticulaire, une resténose après angioplastie, un pontage coronarien, une inflammation, une arthrite, les maladies auto-immunes, un rejet d'allogreffe, les maladies cardio-vasculaires, une maladie fibroproliférative, les maladies provoquées par une agrégation plaquettaire anormale, les maladies provoquées par une prolifération de muscle lisse, un syndrome de Goodpasture, une glomérulonéphrite aiguë, une hypertension pulmonaire néonatale, un asthme, une défaillance cardiaque congestive, une hypertension pulmonaire de l'adulte, une hypertension vasculaire rénale, les rétinopathies prolifératives, une sclérose en plaques, une encéphalo-myélite auto-immune expérimentale, un diabète insulino-dépendant, une maladie intestinale inflammatoire, une recto-colite ulcérative, une maladie de Crohn.

8. Composition pharmaceutique contenant au moins un composé selon la revendication 1 ou 2 en tant qu'ingrédient actif en mélange avec des véhicules et des excipients pharmaceutiquement acceptables.
